Europäisches Patentamt

European Patent Office

Office européen des brevets

(19)

(11) Publication number: **0 270 291**
**A1**

## (12) EUROPEAN PATENT APPLICATION

(21) Application number: **87310342.8**

(22) Date of filing: **24.11.87**

(51) Int. Cl.⁴: **C12Q 1/42 , C12Q 1/44 ,**
**C12Q 1/66 , C12Q 1/26 ,**
**C12Q 1/48**

(30) Priority: **25.11.86 GB 8628108**

(43) Date of publication of application:
**08.06.88 Bulletin 88/23**

(84) Designated Contracting States:
**AT BE CH DE ES FR GB GR IT LI LU NL SE**

(71) Applicant: **London Biotechnology Limited**
**Temple Court 11 Queen Victoria Street**
**London E2N 4TP(GB)**

(72) Inventor: **Rabin, Brian Robert**
**34 Grangewood**
**Potters Bar Hertfordshire, EN6 1SL(GB)**
Inventor: **Hollaway, Michael R.**
**48 Eastwick Road**
**Walton on Thames Surrey, KT12 5AP(GB)**
Inventor: **Harbron, Stuart**
**6 Presburg Road**
**New Malden Surrey, KT3 5AH(GB)**
Inventor: **Benson, Suzanne Marilyn**
**91 Village Way**
**Pinner Middlesex, HA5 5AA(GB)**

(74) Representative: **Armitage, Ian Michael et al**
**MEWBURN ELLIS & CO. 2/3 Cursitor Street**
**London EC4A 1BQ(GB)**

(54) Riboflavin-linked assay procedures and materials.

(57) A method of detecting a phosphatase enzyme which comprises using the enzyme to catalyse the formation of riboflavin from a riboflavin phosphate substrate, enzymically converting the riboflavin to flavin mononucleotide (FMN) and detecting the presence of FMN, preferably by a luminometric procedure. The starting substrate may be FMN, and any unused FMN can be removed by ion exchange to prevent unacceptable interference with the subsequent detection of FMN produced from riboflavin.

EP 0 270 291 A1

## RIBOFLAVIN-LINKED ASSAY PROCEDURES AND MATERIALS

This invention relates to enzyme-linked assays.

Many important non-radioactive diagnostic assays, including the detection and measurement of gene probes and antibodies, involve labelling the detector with an enzyme such as alkaline phosphatase (orthophosphoric monoester hydrolase, EC 3.1.3.1). Several such alkaline phosphatase-labelled systems are available and applications include the detection of pathogenic organisms including viruses, bacteria and fungi; the diagnosis of genetic diseases; and the detection of carriers of genetic diseases.

It is also well known that riboflavin 5'-phosphate (FMN) (Strehler, 1968; Chappelle and Picciolo, 1971; Stanley, 1971; Stanley, 1974; Stanley, 1978; Tsai, 1985) can be conveniently and sensitively determined by a luminometric procedure involving use of enzymes, at present derived from marine organisms, in which light production is catalysed by a luciferase (EC 1.14.14.3) in a reaction (Reaction 2) involving an alkanal and $FMNH_2$ , generated from the analyte FMN by an ancillary enzyme, NAD(P)H:FMN oxidoreductase (EC 1.6.8.1) (Reaction 1), in the presence of a coenzyme, NADH or NADPH.

Reaction 1: $FMN + NAD(P)H + H^+ \rightarrow FMNH_2 + NAD(P)^+$

Reaction 2: $RCHO + FMNH_2 + O_2 \rightarrow RCOOH + FMN + H_2O + h\nu$

According to the present invention there is provided a method of detecting a phosphatase enzyme which comprises using the enzyme to catalyse the formation of riboflavin from a riboflavin phosphate substrate, enzymically converting the riboflavin to flavin mononucloetide (FMN) and detecting the presence of FMN, preferably by a luminometric procedure.

The two enzymic steps are each independently novel, namely:

(i) detecting an enzyme by using it to catalyse the formation of riboflavin from a suitable substrate, and detecting the riboflavin thus produced; and

(ii) detecting riboflavin by enzymically converting it to FMN, and detecting the FMN thus produced by a luminometric procedure.

The use of neither step is considered obvious, in view for example of the ubiquity of FMN and riboflavin, and hence the likelihood of high background signal levels and the consequent apparent unsuitability of these materials as analyte-related components in sensitive assays. The combination of both steps appears even less suitable, since it involves determining the amount of a product which is of the same nature as the starting substrate. Neverthelesss, the results show that the background signal levels can be kept very low, providing an assay of high sensitivity.

Although a large number of assays based on bacterial luminescence have been described, none of these involve the measurement of riboflavin or systems producing riboflavin.

In a preferred procedure riboflavin is produced from riboflavin 5'-phosphate by a reaction which is catalysed by preferably an alkaline phosphatase, phosphatase (Reaction 3).

Reaction 3: $FMN + H_2O \rightarrow Riboflavin + Phosphate$

The phosphatase can itself be the primary analyte, or it can be part of an assay procedure for detecting another analyte.

In the case where the phosphatase substrate would interfere with the subsequent detection, an unused substrate is removed prior to conversion of the riboflavin to FMN. In particular, the use of a short ion-exchange column, anion or mixed bed, has been found to be surprisingly successful in removing unused FMN substrate, to the extent that the riboflavin produced can be detected by the production therefrom of FMN. Other forms of ion-exchange could be used, such as ion exchange paper, or coating the resin on the surface of a tube, or the use of resin-coated beads that can be magneticaly removed.

The invention also includes kits for carrying out the method hereof. A suitable kit may comprise a riboflavin phosphate substrate for the enzyme being detected, enzymic means for converting riboflavin to FMN, and a signal producing system for detecting the presence of FMN, and preferably further includes ion exchange means for removing unused riboflavin phosphate substrate before converting riboflavin to FMN.

A substrate method for converting riboflavin to FMN uses the enzyme ATP: riboflavin 5'-phosphotransferase (EC 2.7.1.26) (Reaction 4).

Reaction 4: $riboflavin + ATP \rightarrow FMN + ADP$

A preferred method of detecting FMN uses a bacterial bioluminescence system. This is proving particularly sensitive, enabling riboflavin to be detected in sub-picomole quantities. Apart from its use in detecting riboflavin produced by alkaline phosphatase-catalysed conversion from FMN or the like, this system (ii) can be coupled to other assay reactions leading to the generation of riboflavin, such as ribonuclease acting on an appropriate substrate, as illustrated futher in GB 2156518A.

Instead of using a luminometric assay to detect the FMN, it can form part of an enzyme-linked signal amplification system, such as described in our GB 2156518A, where for example the FMN leads to the production of a holoenzyme from an apoenzyme, which in turn catalyses a signal-producing reaction. One example would be the formation of flavodoxin from FMN and apoflavodoxin; the flavodoxin then mediating the reductase catalysed reduction of cytochrome c by NADPH. In another example the FMN could convert apopyridoxol phosphate oxidase (EC 1.4.3.5) to the holoenzyme which catalyses the conversion of pyridoxol phosphate to pyridoxal phosphate, the latter converting apoaspartate transaminase to the holoenzyme, initiating a chain of enzymic reactions leading to the formation of a colour signal.

The efficacy of the procedures of this invention are illustrated by the following example.

Alkaline phosphatase catalysed production of riboflavin

A typical incubation mixture for the assay of alkaline phosphatase is given below:

```
10   mM    Tris-acetate buffer, pH 8.0

 1   mM    Mg2+

 1   mM    FMN

           Alkaline phosphatase in varying amounts

           (commercial preparation from Calzyme

           Laboratories Inc., Lot 25-4-31)


           Total volume 0.2 ml
```

After a period of incubation (at 25°C) the reaction mixture is loaded onto a 0.5 ml DEAE-cellulose column (DE52 Whatman, 8 mm x 9 mm dia) equilibrated with the Tris-acetate buffer and washed through with 1.0 ml of the same buffer to elute the riboflavin and retain unreacted FMN, giving a final volume of 1.2 ml.

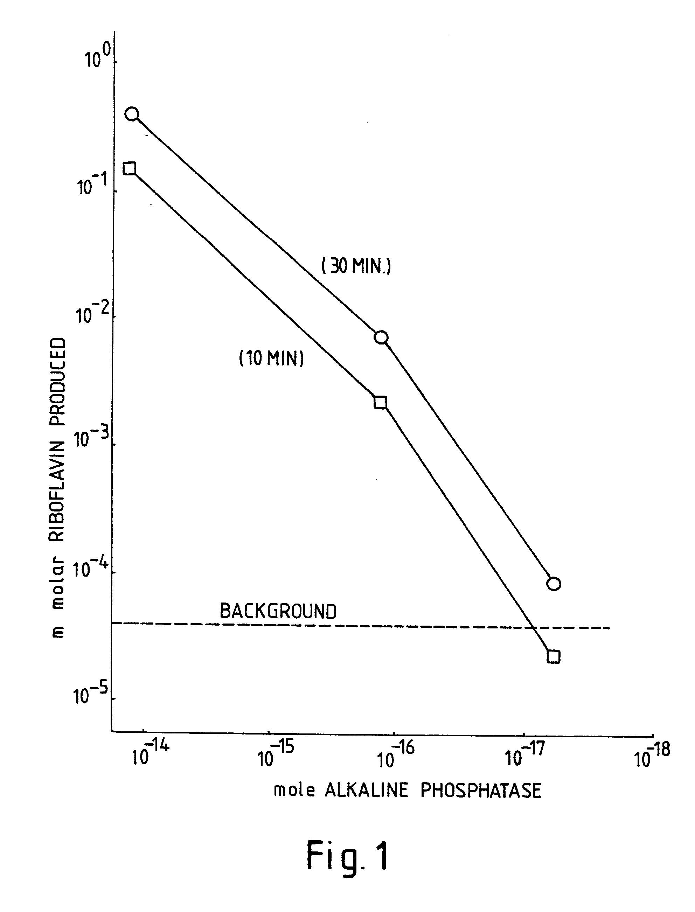

The amount of riboflavin produced is measured luminometrically as described below. Fig. 1 shows a graph of the amount of riboflavin produced for different amounts of alkaline phosphatase, given two different incubation times of 10 minutes and 30 minutes respectively. The amount of alkaline phosphatase given represents the amount in 0.07 ml used in the luminometric assay (from the 1.2 mL eluted), whilst the concentration of riboflavin given refers to the concentration in the incubation mixture after subtraction of the background.

Estimation of Riboflavin

A typical mixture for the assay of riboflavin is given below:

| 10 | mM | phosphate buffer, pH 7.0 |
|---|---|---|
| 0.1 | mM | dithiothreitol |
| 0.1 | mM | NADH |
| 0.007 | mM | decylaldehyde |
| 1 | mg/ml | Triton X-100 |
| 0.04 | mg/ml | luciferase (purified commercial preparation from BCL) |
| 10 | U/ml | reductase (purified commercial preparation from BCL) |
| 0.1 | mM | ATP |
| 0.05 | mM | $ZnSO_4$ |
| 0.035 | mg/ml | riboflavin kinase (prepared by the method of Merrill & McCormick, 1980) |
| 0.07 | ml | riboflavin solution (from the foregoing procedure). |

Using a commercial luminometer (eg LKB 1250) the background level of light emission is measured. Riboflavin is then added and the increase in light emission noted.

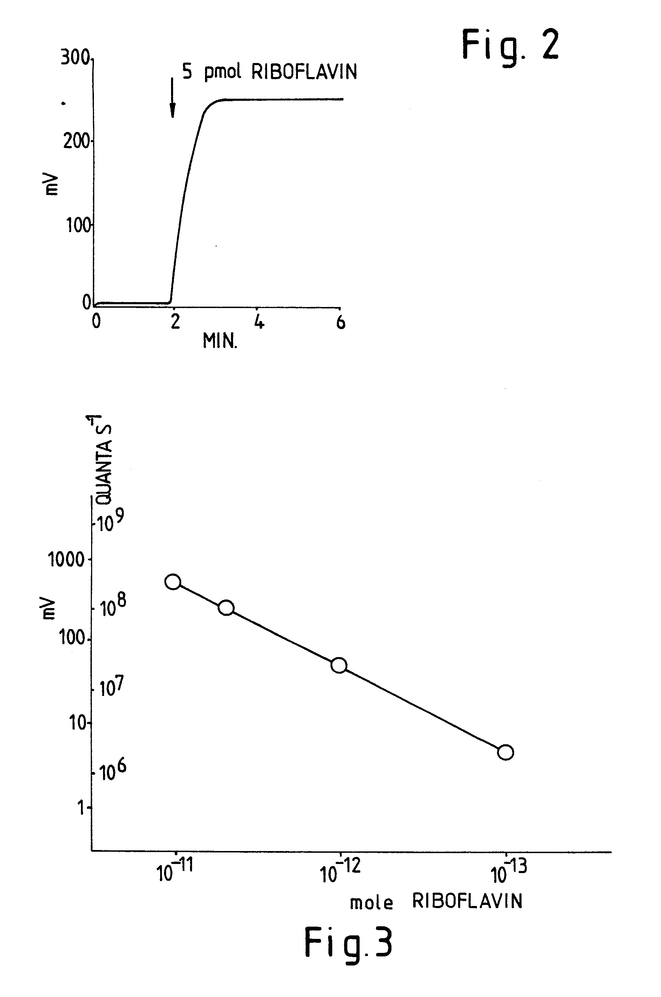

Fig. 2 shows a trace of the output from the luminometer during the assay of 5 pmol of riboflavin. The signal produced remained constant for more than 30 minutes. The low signal level for the first 2 minutes before adding the riboflavin indicates the background signal; about 2-3 mV. The addition of 5 pmol of riboflavin raises the signal by a factor of about 100.

By repeating the experiment using different amounts of riboflavin from 100 fmol to 10 pmol a standard curve was constructed as shown in Fig. 3. From the standard curve the quantity of riboflavin in an unknown sample may be very quickly and easily obtained.

It also shows that riboflavin levels can be measured down to about 100 fmol ($10^{-13}$ mol) before the background signal becomes too severe. To achieve that low level of background, purification was carried out beforehand on the luciferase and reductase enzymes and also the FMN substrate for the alkaline phosphatase.

The luciferase and reductase enzymes contained detectable amounts of FMN. Accordingly the enzymes were purified by ion exchange chromatography by FPLC (MonoQ HR 10/10, Pharmacia), using 20 mM bis-Tris propane at pH7 as the buffer, and eluting with 350 mM NaCl in the same buffer (a gradient of 2.2 mM/ml). The chromatography was repeated three times.

The starting substrate FMN contained a substantial amount of riboflavin (up to about 12% is normal in commercial samples). The FMN was therefore purified by the same procedure as described above, except that it was eluted with up to 700 mM ammonium acetate in the buffer (a gradient of 4.4 mM/ml). A single pass was enough to remove riboflavin to a low level. The fractions were pooled and freeze-dried.

Control samples, without alkaline phosphatase, were assayed as described above, and the background amount of riboflavin passing through the column show to be less than 25 picomole.

Clearly, further purification of either the enzymes or the FMN, or both, can be expected to reduce the background signal further, enabling a sensitivity to be achieved which approaches that of radiochemical procedures. Even at the present background levels, the assay is better than any currently available commercial method (radiochemical assays being in general unsuitable for other than laboratory work).

An approximate kinetic analysis from the foregoing results gives a value for the $k_{cat}$ of the enzyme with the FMN of about 580 s$^{-1}$ and a value for the $K_m$ of about 0.9 mM.

The luciferase and the reductase enzymes could if desired be co-immobilised on a suitable support. Also, the light produced could be detected photographically instead of by a luminometer.

## REFERENCES

Chappelle E.W. and Picciolo G.L. (1971)
Assay of flavin mononucleotide (FMN) and flavin adenine dinucleotide (FAD) using the bacterial luciferase reaction. Methods in Enzymology 18, 381-385.

Merrill A.H. and McCormick D.B. (1980)
Affinity chromatographic purification and properties of flavokinase (ATP: Riboflavin 5'-phosphotransferase) from rat liver. J. Biol. Chem. 255, 1335-1338.

Stanley P.E. (1971)
The use of the liquid scintillation spectrometer for measuring NADH and FMN by the Photobacterium luciferase and ATP by the firefly luciferase. In: Organic Scintillators and Liquid Scintillation Counting - (Horrocks D.L. and Peng C.T., eds.), pp. 607-620. Academic Press, New York.

Stanley P.E. (1974)
Analytical bioluminescent assays using the liquid scintillation spectrometer. A review in Liquid Scintillation Counting (Crook M.A. and Johnson P., eds), Vol. 3, pp 253-272. Heyden, London.

Stanley P.E. (1978)
Quantitation of picomole amounts of NADH, NADPH, and FMN using bacterial luciferase. Methods in Enzymology 57, 215-222.

Strehler B.L. (1968)
Bioluminescence Assay: Principles and Practice. Methods of Biochemical Analysis 16, 99-181.

Tsai T.S. (1985)
Flavin mononucleotide. Methods of Enzymatic Analysis (3rd edition) 7, 304-313.

## Claims

1. A method of detecting a phosphatase enzyme which comprises using the enzyme to catalyse the formation of riboflavin from a riboflavin phosphate substrate, enzymically converting the riboflavin to flavin mononucleotide (FMN) and detecting the presence of FMN.

2. A method according to claim 1 wherein any unused substrate is removed by ion exchange before converting the riboflavin to FMN.

3. A method according to claim 2 wherein riboflavin is produced from FMN.

4. A method according to any one of claims 1, 2 and 3 wherein the said enzyme is the primary analyte to be detected.

5. A method according to any one of claims 1, 2 and 3 wherein said enzyme is part of an assay procedure to determine another substance which is the primary analyte to be detected.

6. A method according to any one of the preceding claims wherein the FMN is detected by means of an enzyme-linked signal amplification system.

7. A method according to claims 6 wherein the FMN leads to the production of a holoenzyme from an apoenzyme, which in turn catalyses a signal-producing reaction.

8. A method according to any one of claims 1 to 5 wherein the FMN is detected by means of a luminometric procedure.

9. A method according to claim 8 wherein a bacterial bioluminescence system is used to detect FMN.

10. A method according to claim 9 wherein the bioluminescence involves using the enzyme luciferase (EC 1.14.14.3) and a system generating $FMNH_2$ from FMN.

f11. A method according to claim 10 wherein the system generating $FMNH_2$ is NAD(P)H: FMN oxidoreductase (EC 1.6.8.1) plus NADPH or NADH.

12. A method according to claim 10 wherein the system generating $FMNH_2$ uses a diaphorase enzyme plus NADH.

13. A method according to any one of the preceding claims wherein the phosphatase enzyme is an alkaline phosphatase.

14. A method according to any one of the preceding claims wherein riboflavin is converted to FMN by the enzyme ATP: riboflavin 5'-phosphotransferase. (EC 2.7.1.26).

15. A kit for carrying out an assay of claim 1, which includes a riboflavin phosphate substrate for the enzyme being detected, enzymic means for converting riboflavin to FMN, and a signal producing system for detecting the presence of FMN.

16. A kit according to claim 15 which further includes ion exchange means for removing unused riboflavin phosphate substrate before converting riboflavin to FMN.

Fig. 1

0 270 291

Fig. 2

Fig.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 156 641 (UNIVERSITY COLLEGE LONDON) * Pages 6-8,11,17; page 21, lines 17-20; page 29, line 20 - page 30, line 19 * & GB-A-2 156 518 (Cat. D) | 1,3-11, 13,15 | C 12 Q 1/42 C 12 Q 1/44 C 12 Q 1/66 C 12 Q 1/26 C 12 Q 1/48 |
| A | | 12,14 | |
| Y | EP-A-0 156 204 (KABUSHIKI KAISHA TOSHIBA) * Page 7, line 32 - page 8, line 28 * | 8-11,15 | |
| Y | WO-A-8 503 356 (MOLECULAR BIOSYSTEMS INC.) * Whole document * | 4-11,15 | |
| Y | CHEMICAL ABSTRACTS, vol. 98, no. 18, 2nd May 1983, page 409, abstract no. 159456y, Columbus, Ohio, US; H. DANIEL et al.: "Hydrolysis of FMN and FAD by alkaline phosphatase of the intestinal brush-border membrane", & INT. J. VITAM. NUTR. RES. 1983, 53(1), 109-14 * Abstract * | 1,3,13 | TECHNICAL FIELDS SEARCHED (Int. Cl.4) C 12 Q |
| A | CHEMICAL ABSTRACTS, vol. 83, no. 17, 27th October 1975, page 177, abstract no. 143427u, Columbus, Ohio, US; G.M. SHAVLOVSKII et al.: "Determination of riboflavine kinase activity in yeasts", & UKR. BIOKHIM. ZH. 1975, 47(4), 536-41 * Abstract * ---  -/- | 1,3,14 | |

The present search report has been drawn up for all claims

| Place of search | Date of completion of the search | Examiner |
|---|---|---|
| THE HAGUE | 12-02-1988 | GRIFFITH G. |

**European Patent Office**

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| A | CHEMICAL ABSTRACTS, vol. 66,. 1967, page 7842, abstract no. 83869b, Columbus, Ohio, US; J. PONTINEN et al.: "Hydrolysis of riboflavine 5'-phosphate by phosphatases of rat kidney", & HISTOCHEMIE 8, 283-7, 1967 <br> * Abstract * <br> ----- | 1,3,13 | |

TECHNICAL FIELDS
SEARCHED (Int. Cl.4)

The present search report has been drawn up for all claims

| Place of search | Date of completion of the search | Examiner |
|---|---|---|
| THE HAGUE | 12-02-1988 | GRIFFITH G. |

CATEGORY OF CITED DOCUMENTS

X : particularly relevant if taken alone
Y : particularly relevant if combined with another
 document of the same category
A : technological background
O : non-written disclosure
P : intermediate document

T : theory or principle underlying the invention
E : earlier patent document, but published on, or
 after the filing date
D : document cited in the application
L : document cited for other reasons
...............................................................................
& : member of the same patent family, corresponding
 document

EPO FORM 1503 03.82 (P0401)